# EUROPEAN PATENT SPECIFICATION

(11) **EP 1 430 061 B1**
(45) Date of publication and mention of the grant of the patent: **09.11.2011**
(21) Application number: 02765666.9
(22) Date of filing: 18.09.2002
(51) Int. Cl.: C07F 5/00

(54) **INCLUSION COMPOUND COMPRISING CUCURBITURIL DERIVATIVES AS HOST MOLECULE AND PHARMACEUTICAL COMPOSITION COMPRISING THE SAME**
EINSCHLUSSVERBINDUNG MIT CUCURBITURILDERIVATEN ALS WIRTSMOLEKÜL, UND DIESE ENTHALTENDE PHARMAZEUTISCHE ZUSAMMENSETZUNG
COMPOSE D'INCLUSION CONTENANT DES DERIVES DE CUCURBITURIL EN TANT QUE MOLECULE RECEPTRICE ET COMPOSITION PHARMACEUTIQUE LES CONTENANT

(30) Priority: 18.09.2001 KR 2001057573
(43) Date of publication of application: 23.06.2004
(73) Proprietor: Postech Foundation, Pohang-City, Kyungsangbuk-do (KR)
(72) Inventor: KIM, Kimoon, Pohang Univ. of Science and Techn., Pohang-shi, 790-330 Kyungsangbuk-do (KR); JEON, Young Jin, Pohang Univ. of Science & Techn., Pohang-shi, 790-330 Kyungsangbuk-do (KR); KIM, Soo-Young, Pohang Univ. of Science and Techn., Pohang-shi, 790-330 Kyungsangbuk-do (KR); KO, Young Ho, Pohang Univ. of Science and Techn., Pohang-shi, 790-330 Kyungsangbuk-do (KR)
(74) Representative: Stanley, David William
(86) International application number: PCT/KR2002/001755
(87) International publication number: WO 2003/024978

(56) References cited:
- EP-A- 1 094 065
- US-A- 5 965 118
- BIOGIONI MARIO ET AL.: 'Electronic attack of (I(py)2)+(NO3-) on three-coordinate Pt0 precursors: Synthesis and in vitro antitumor activity of water-soluble, five-coordinate Pt2 complexes' EUROPEAN J. OF INORGANIC CHEMISTRY vol. 2000, no. 8, 2000, WILEY-VCH VERLAG GMBH, 2000, pages 1717 - 1721, XP008092719
- DE OLIVEIRA M.B. ET AL.: 'New perfluorophthalate complexes of platinum(II) with chemotherapeutic potential' METAL-BASED DRUGS vol. 3, no. 3, 1996, FREUND, 1996, pages 117 - 122, XP008093343
- BEVERLY A. TEICHER ET AL.: 'Combination of etanidazole with cyclophosphamide and platinum complexes' CANCER CHEMOTHERAPY AND PHARMACOLOGY vol. 28, no. 3, 1991, pages 153 - 158, XP008092243
- KIM UI-RAK ET AL.: 'The study about interation of cis-diaminedichloroplatin(cis-DDP) complexes with DNA base, 1-methylcytosine, for development of anti-tumor drugs' TAEHAN HWAHAKHOE CHI vol. 34, no. 4, 1990, pages 331 - 339, XP008095077

## Description

### Technical Field

The present invention relates to an inclusion compound and pharmaceutical composition containing the same,

### Background Art

Numerous anticancer agents using platinum complexes have been synthesized since the discovery of the anticancer effect of platinum complexes. However, the anticancer agents hitherto synthesized have not been satisfactory in view of pharmaceutical efficacy and remain open for improvement in their toxicity and solubility.

Currently, cisplatin which is known as one of effective anticancer agents that are widely used, is particularly effective for treatment of ovarian cancer or testicular cancer and is very contributable to treatment of various kinds of cancers. However, since cisplatin has extremely poor water-soluble and organic-soluble properties in itself, it cannot be easily administered, suggesting limitation in the use thereof. Also, cisplatin disadvantageously has serious toxicity. Attempts to overcome such drawbacks of cisplatin, various anticancer agents are under development, and several thousands of anticancer agents have been synthesized. Some of the synthesized anticancer agents are under clinical trials.

Carboplatin is less toxic than cisplatin, and can be administered to patients in a larger amount, that is, approximately of 2000 mg/dose. However, carboplatin is only effective in treatment of tumor cells resistant to cisplatin, and can only be administered intravenously.

Recently, two platinum complexes have been restrictively authorized as anticancer agents; (trans-L-diaminocyclohexane)oxalatoplatinum (II) (oxaliplatin or L-OHP); and cis-diammine-glycoloato-O,Ó-platinum (II) (nedaplatin or 254-S). The former platinum complex is currently being used for secondary treatment of metastatic colorectal cancer in Japan and France, while the latter one is authorized to be commercially available in Japan.

However, in clinical testing, oxaliplatin or nedaplatin demonstrated no distinctive efficacy than cisplatin or carboplatin. Only oxaliplatin exhibited potentiality to be used for treatment of tumors resistant to cisplatin in all clinical trials. Research into platinum complexes having enhanced anticancer effects is continuously carried out, with the aim of development of platinum complexes that are less toxic, capable of administrating orally and free of cross resistance to cisplatin and carboplatin.

Existing platinum complex anticancer agents have limitation in improvement of pharmaceutical efficacy by modifying ligand bonded to platinum, and have much room for improvement in view of chemical stability and oral administration.

A novel inclusion compound having 1, 1-cyclobutanedicarboxylated diamine platinum (II) (also called "carboplatin") included in alpha-cyclodextrine has been reported. This compound is characterized in that it has a function of increasing water solubility of a platinum complex used as an anticancer agent. However, since a binding constant between alpha-cyclodextrine and carboplatin is low, the binding energy between the two compounds. The inclusion compound is known to have little difference in anticancer effect compared to the case of using only carboplatin.

EP-1094065A discloses a 2:1 complex of cis dichloroethylenediamine platinum (II) and cucurbituril.

Platinum-based antitumor drugs are disclosed in Wong, E. et al, Chem. Rev (99) 9, pages 2451-2466(1999).

### Disclosoure of the Invention

An object of the present invention is to provide pharmaceutical compositions containing inclusion compounds having metal complexes such as platinum complexes as a guest molecule, and preparation methods thereof.

The present invention provides a pharmaceutical composition comprising an inclusion compound having a cucurbituril derivative of the formula 1 as a host molecule and a metal complex selected from the group consisting of compounds represented by formulas 4 through 27 and *cis-*dichloroethylenediamine platinum (II) as a guest molecule: 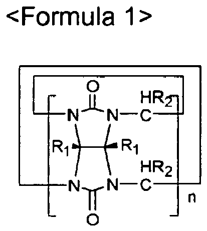 wherein R₁ and R₂ are independently selected from the group consisting of H, C₁-C₃₀ alkyl, C₁-C₃₀ alkene, C₁-C₃₀ alkyne, C₁-C₃₀ alkylthio, C₁-C₃₀ alkylcarboxy, C₁-C₃₀ alkylhydroxy, C₁-C₃₀ alkylsilyl, C₁-C₃₀ alkyloxy, C₁-C₃₀ haloalkyl, nitro, C₁-C₃₀ alkylamine, amine, C₅-C₃₀ unsubstituted cycloalkyl or C₅-C₃₀ cycloalkyl having a hetero atom, and C₆-C₁₅ unsubstituted aryl or C₆-C₁₅ aryl having a hetero atom, n is an integer from 4 to 20,

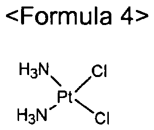

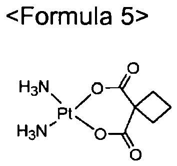

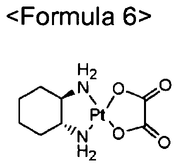

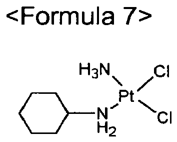

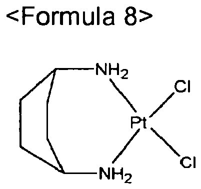

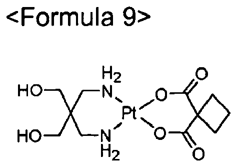

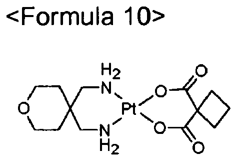

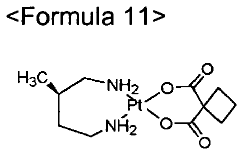

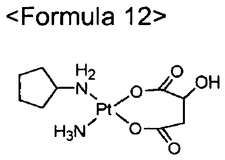

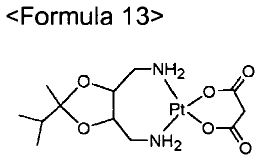

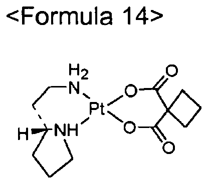

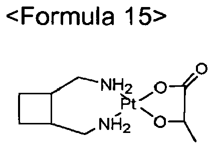

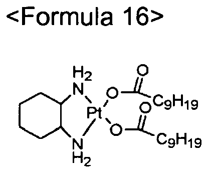

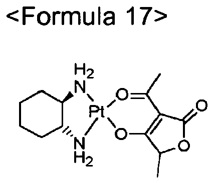

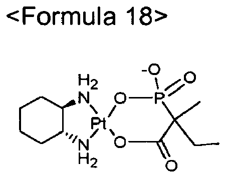

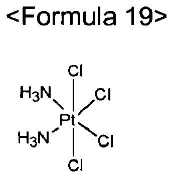

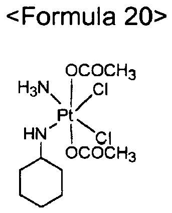

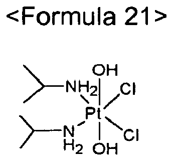

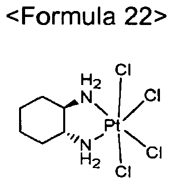

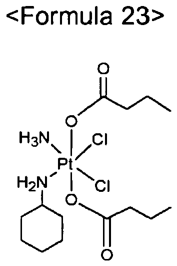

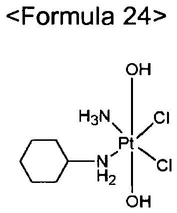

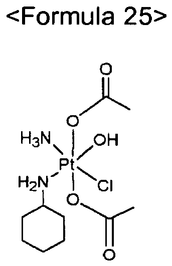

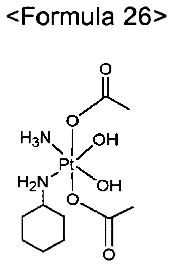

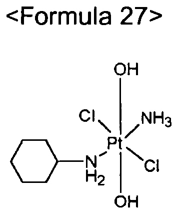

In the inclusion compound, the binding ratio of the compound of the formula 1 to the metal complex is preferably in the range of 1:1 to 1:8 or 1:4. In the compound of the formula 1, R, may be hydrogen, methyl, ethyl, propyl, isopropyl, butyl, isobutyl, phenyl or pyridyl; R₂ may be hydrogen, propyl, phenyl, trichloromethyl, trifluoromethyl, parafluoromethyl or α, α, α-trifluorotoluyl, and n is preferably an integer from 5 to 8.

According to another aspect of the present invention, there is provided a method of preparing a pharmaceutical composition according to any of the preceding aspects of the invention, comprising the step of reacting the cucurbituril derivative of the formula 1 with the metal complex selected from the group consisting of compounds represented by formulas 4 through 27 and *cis-*dichloroethylenediamine platinum (II).

The reacting step may be performed at room temperature in the presence of a water solvent and includes adding an alcoholic solvent to the resultant product to separate a precipitate. Or, the reacting step may be performed at a hydrothermal reactor at a temperature of 80 to 200°C, followed by cooling down to room temperature to separate a crystalline material.

In a pharmaceutical composition according to any of the preceding aspects of the invention, the metal complex may be an anticancer active component and the inclusion compound may be used for treatment of cancer.

In a pharmaceutical composition according to any of the preceding aspects of the invention, the inclusion compound is used for treatment of ovarian cancer, breast cancer or colonic cancer.

An inclusion compound for use in the present invention may be represented by the formula 3, wherein m is an integer from 1 to 8.

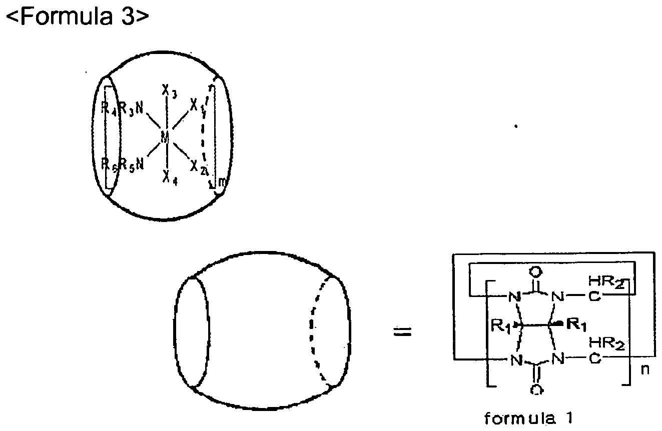

The inclusion compound of the formula 3 is preferably maintained at a stable state such that hydrogen atoms bonded to nitrogen atoms included in the metal complex of the formula 2 form hydrogen bonds with oxygen atoms of the cucurbituril derivative, and hydrophobic substituents bonded to nitrogen atoms in the metal complex of the formula 2 are positioned in hydrophobic cavities.

The prepareation of the cucurbituril derivative of the formula 1 was improved by the applicant of the present invention to synthesize and separate cucurbit[6]uril and its homologue cucurbitu[n]uril (n=5, 7 and 8), which was confirmed by X-ray crystal structure determination (U.S. Patent No. 6,365,734). According to this procedure, unlike the conventional separation method in which only hexametric cucurbituril was separated, pentametric, heptametric and octametric cucurbiturils can be separated, thereby selecting a host molecule according to the size of a guest molecule.

In the formulas 1 and 2, examples of the C₁-C₃₀ alkyl in R₁, R₂, R₃, R₄, R₅ and R₆, include methyl, ethyl, propyl, isopropyl and tert-butyl, examples of the C₁-C₃₀ alkenyl include propylene and butene, examples of the C₁-C₃₀ alkynyl include hexynyl, examples of the C₁-C₃₀ alkylthio include butylmethyl sulfide and octanethiol, examples of the C₁-C₃₀ alkylcarboxyl include carboxypropyl and carboxybutyl, examples of the C₁-C₃₀ hydroxyalkyl include hydroxybutyl and hydroxyethyl, examples of the C₁-C₃₀ alkylsilyl include allyltriethylsilyl and vinyltriethylsilyl, examples of the C₁-C₃₀ alkoxy include methoxy and ethoxy, examples of the C₁-C₃₀ haloalkyl include CF₃ and CH₂Cl, examples of the C₁-C₃₀ aminoalkyl include 2-aminobutyl and 1-aminobutyl, examples of the C₅-C₃₀ unsubstituted cycloalkyl include cyclohexyl and cyclopentyl, examples of the C₅-C₃₀ cycloalkyl having a hetero atom include piperidyl and tetrahydrofuranyl, examples of C₆-C₃₀ unsubstituted aryl include phenyl, benzyl and naphthyl, and examples of C₆-C₃₀ aryl having a hetero atom include pentafluorophenyl or pyridyl. In X₁, X₂, X₃ and X₄, examples of the halogen atom include Br, Cl, I and F, examples of the C₁-C₃₀ haloalkyl include bromomethyl and chloromethyl, examples of the C₁-C₃₀. alkylcarboxyl include CH₃C(=O)O-, and example of C₁-C₃₀ alkyldicarboxy include oxalato and malonato.

In M, examples of the transition metal include Pt, Pd and Au, examples of the lanthanide metal include Ln, Gd and Ce, examples of the actinide metals include Ac, examples of the alkali metal include Li, Na and K, and examples of the alkali earth metal include Mg and Ca.

The cucurbituril derivatives of the formula 1 is good in solubility in general solvents. In the cucurbituril derivatives of the formula 1, in particular, R₁ is preferably methyl, ethyl, propyl, isopropyl, butyl, isobutyl, phenyl or pyridyl, R₂ is preferably hydrogen, propyl, phenyl, trichloromethyl, trifluoromethyl, parafluoromethyl or α, α, α -trifluorotoluyl, and n is preferably an integer from 5 to 8. In cucurbituril derivatives of the formula 1, it is more preferable that R₁ and R₂ are both hydrogen and n is in the range from 5 to 8. These compounds have a water-solubility of 1×10⁻¹ to 3×10⁻¹ M, and have a good solubility in an organic solvent, in particular, in one selected from the group consisting of methanol, ethanol, dimethylsulfoxide, dimethylformamide and acetonitrile, that is, 1×10⁻⁴ to 1×10⁻² M.

In the case where M is a divalent platinum ion, and X₃ and X₄ denote non-bonding, examples of the complex of the formula 2 include cisplatin of the formula 4 wherein X₁ and X₂ are both Cl, and R₃ through R₈ are all H, carboplatin of the formula 5 wherein X₁ and X₂ are both Cl, and X₃ and X₄ are represented by the structural formula a, and R₃ through R₈ are all H, oxaliplatin of the formula 6 wherein R₃, R₄, R₆ and R₇ are all H, R₅ and R₆ are represented by the structural formula j, and X₁ and X₂ are represented by the structural formula b, JM118 of the formula 7 wherein X₁ and X₂ are both Cl, R₃, R₄, R₅, R₇ and R₈ are all H, and R₆ is represented by the structural formula j, Pt(cis-1,4-dach)Cl₂, of the formula 8 (Here, dach represents 1,2-diaminocyclohexyl or 1,2-diaminocyclohexane) wherein X₁ and X₂ are both Cl, R₃, R₄, R₇ and R₈ are all H, and R₅ and R₆ are interconnected to each other and represented by the structural formula i, xeniplatin of the formula 9 wherein X₁ and X₂ are both Cl, R₃, R₄, R₇ and R₈ are all H, and R₅ and R₆ are represented by the structural formula k, enloplatin of the formula 10 wherein X₁ and X₂ are interconnected to each other and represented by the structural formula a, R₃, R₄, R₇ and R₈ are all H, and R₅ and R₆ are interconnected to each other and represented by the structural formula ℓ , Cl-973 of the formula 11 wherein X₁ and X₂ are interconnected to each other and represented by the structural formula m, R₃, R₄, R₇ and R₈ are all H, and R₅ and R₆ are interconnected to each other and represented by the structural formula a, cycloplatam of the formula 12 wherein X₁ and X₂ are interconnected to each other and represented by the structural formula f and R₃, R₄, R₆, R₇ and R₈ are all H, and R₅ is cyclopentyl, SKI 2053R of the formula 13 wherein X₁ and X₂ are interconnected to each other and represented by the structural formula c, R₃, R₄, R₇ and R₈ are all H, and R₅ and R₆ are represented by the structural formula n, miboplatin of the formula 14 wherein X₁ and X₂ are interconnected to each other and represented by the structural formula a, R₃, R₄ and R₈ are all H, and R₅, R₆ and R₇ are represented by the structural formula o, iobaplatin of the formula 15 wherein X₁ and X₂ are interconnected to each other and represented by the structural formula g, R₃, R₄, R₇ and R₈ are all H, and R₅ and R₆ are represented by the structural formula p, L-NDDP of the formula 16 wherein X₁ and X₂ are both C₉H₁₉, R₃, R₄, R₇ and R₈ are all H, and R₅ and R₆ are interconnected to each other and represented by the structural formula j, TRK-710 of the formula 17 wherein X₁ and X₂ are interconnected to each other and represented by the structural formula h, R₃, R₄, R₇ and R₈ are all H, and R₅ and R₆ are interconnected to each other and represented by the structural formula j, and Na[Pt(R,R-dach)(MPBA)] of the formula 18 wherein X₁ and X₂ are interconnected to each other and represented by the structural formula d, R₃, R₄, R₇ and R₈ are all H, and R₅ and R₆ are interconnected to each other and represented by the structural formula j.

In the case where X₃ and X₄ represent various kinds of substituents, examples of the of the complex of the formula 2 include cisdiaminedichloroplatinium (IV) of the formula 19 wherein X₁, X₂, X₃ and X₄ are all Cl, R₃, R₄, R₅, R₆, R₇ and R₈ are all H, JM216 of the formula 20 wherein X₁ and X₂ are both Cl, X₃ and X₄ are both OC(=O)CH₃, R₃, R₄, R₅, R₇ and R₈ are all H, and R₆ are cyclohexyl, iprolatin of the formula 21 wherein X₁ and X₂ are both Cl, X₃ and X₄ are both OH, R₃, R₄, R₇ and R₈ are all H, R₅ and R₆ are both isopropyl, omaplatin of the formula 22 wherein X₁, X₂, X₃ and X₄ are all Cl, R₃, R₄, R₅, R₇ and R₈ are all H, and R₅ and R₆ are interconnected to each other and represented by the structural formula j, JM221 of the formula 23 wherein X₁ and X₂ are both Cl, X₃ and X₄ are both -OC(=O)CH₂CH₂CH₃, R₃, R₄, R₅, R₇ and R₈ are all H, and R₆ is cyclohexyl, JM149 of the formula 24 wherein X₁ and X₂ are both Cl, X₃ and X₄ are both OH, R₃, R₄, R₅, R₇ and R₈ are all H, and R₆ is cyclohexyl, JM518 of the formula 25 wherein X₁ is OH, X₂ is Cl, X₃ and X₄ are both -OC(=O)CH₃, R₃, R₄, R₅, R₇ and R₈ are all any R₆ is cyclohexyl, JM383 of the formula 26 wherein X₁ and X₂ are both OH, X₃ and X₄ are both -OC(=O)CH₃, R₃, R₄, R₅, R₇ and R₈ are all H, and R₆ is cyclohexyl, and JM335 of the formula 27 wherein X₁ is NH₃, X₂ is Cl, X₃ and X₄ are both OH, R₃, R₄, Ras, R₇ and R₈ are both H, and R₆ is cyclohexyl.

Preparation methods of the inclusion compounds of the formula 3 according to the present invention will now be described.

The inclusion compounds of the formula 3 can be obtained by mixing the cucurbituril derivatives of the formula 1 with the metal complexes of the formula 2, followed by stirring and reacting.

The reaction is carried out by stirring the reactants at room temperature for 1 to 6 hours in the presence of water as a solvent, or reacting in a hydrothermal reactor at 80 to 200 °C, in particular, 100 to 120°C, for 1 to 3 days and then being allowed to stand at 50 to 70°C for 1 to 2 days.

During the hydrothermal reaction, the reaction is preferably carried out in the above-noted temperature range in view of reactivity. A work-up procedure resulting from the reaction will now be briefly described. After reacting at room temperature, an alcoholic solvent such as methanol or ethanol is added to the reaction product to separate the resultant in the form of precipitate. During the reaction carried out at the hydrothermal reactor, the reaction product is cooled to room temperature to then precipitate in a crystalline form.

In preparing the inclusion compounds of the formula 3, the binding ratio of the cucurbituril derivative of the formula 1 to the metal complex of the formula 2 can be adjusted according to equivalents in consideration of sizes of cavities of cucurbituril. Preferably, 1 to 10 quivalents of the metal complex of the formula 2 is used based on one equivalent of the cucurbituril derivative of the formula 1.

The synthesizing methods of the cucurbituril derivatives of the formula 1, as described in U.S. Patent No. 6,365,734 to the applicant of the present invention, will now be described in more detail.

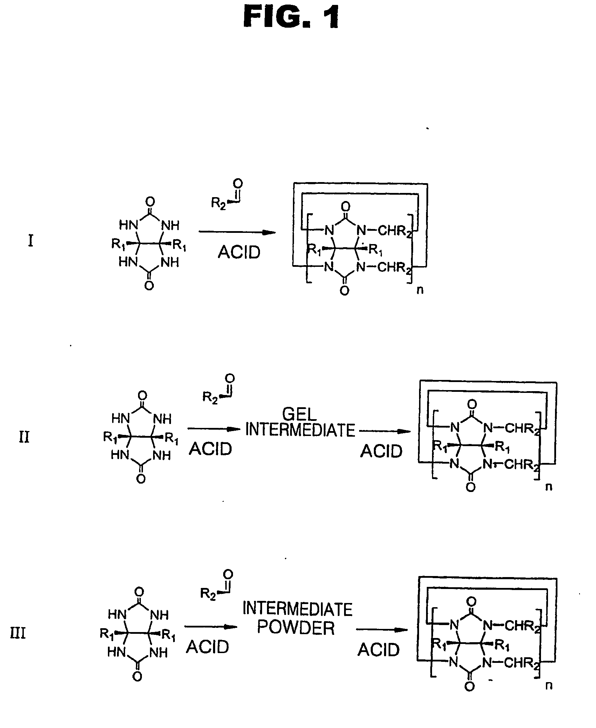

The method for synthesizing the cucurbituril derivatives according to the present invention can be classified into one of three methods, according to the reaction conditions and the state of intermediate products (refer to FIG. 1).

First, an acid is added to glycoluril in an amount of 3 to 7 moles with respect to 1 mole of glycoluril, and mixed. Preferably, the acid is preferably diluted with water or an organic solvent to be 6 to 12 M. Any acid capable of dissolving glycoluril, for example, hydrochloric acid, sulfuric acid, phosphoric acid, acetic acid, nitric acid and an mixtures of these acids, can be used. The organic solvent as an acid diluent may be dimethylsulfoxide, N,N-dimethylformamide, methanol, ethanol, chloroform or an mixture of these solvents.

The glycoluril can be synthesized by the following method, and can be purchased.

Urea and glyoxal are dissolved in an aqueous acidic solution or an acid-containing organic solvent, and stirred for a certain period of time. Removal of water or the organic solvent from the reaction mixture produces glycoluril.

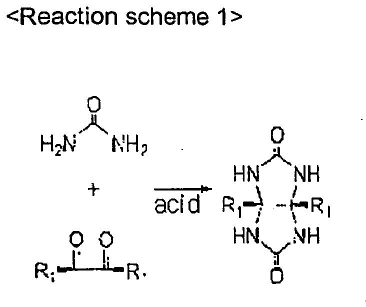

Formaldehyde is added to a mixture of the glycoluril and acid for reaction while stirring at 70 to 95°C for 6 to 24 hours. The amount of formaldehyde used is 2 to 20 moles for each mole of glycoluril, but preferably, 4 moles. During the reaction, the color of the reaction solution changes into dark red with time.

The reaction mixture is subjected to a further reaction at 95 to 105°C. The final reaction product varies depending on the reaction temperature and the amount of reactants. The usual final reaction product is a mixture of two or more cucurbituril derivatives, where n is a value from 5 to 20.

Typically, the reaction product is a mixture of 5 to 30% of the cucurbituril derivative with n=5, 30 to 70% of the cucurbituril derivative with n=6, 5 to 30% of the cucurbituril derivative with n=7, 2 to 15% of the cucurbituril derivative with n=8, and 1 to 10% of the cucurbituril derivatives with n=9 to 20.

However, during the reaction at 95 to 105°C, the cucurbituril derivative with n=6 may precipitate in a crystalline form, depending on the reaction temperature, moisture content in the air, and concentration of reactants.

Then, the resulting cucurbituril derivatives are separated from each other by the following fractional crystallization procedure, which will now be described in more detail.

First, the final reaction mixture is diluted with water and left on a bench at room temperature to give the cucurbituril derivative having the formula (1), where n=8. During this separation step, the cucurbituril derivative with n=6 may be formed in a crystalline form, and can be easily separated from the cucurbituril derivative with n=8 in the formula (1), with a solvent. The solvent for use in the separation of the cucurbituril derivative with n=6 may be an alkali metal ion salt solution, such as Na₂SO₄ or K₂SO₄, an amine-acid salt solution (H₂N-R-NH₂.2HCl) or a formic acid solution. The crystalline cucurbituril derivative with n=6 is soluble in such a solvent, whereas the cucurbituril derivative with n=8 is relatively less soluble in the solvent, which enables the separation of the two reaction products.

After separation of the cucurbituril derivative with n=8 in the formula (1), the filtrate is further diluted with water and acetone, and filtered. Preferably, water and acetone are added in a ratio of 1:3 to 1:7 by volume. The filtrate after the filtration contains the cucurbituril derivatives having the formula (1), where n=9 to 20. Meanwhile, the filter cake is dissolved in water. Here, the cucurbituril derivatives with n=5 and 7 in the formula (1) are obtained as a major component of the water soluble fraction. The major component in the water insoluble fraction is the cucurbituril derivative with n=6 in the formula (1).

The cucurbituril derivatives with n=5 and 7 in the formula (1) can be separated from each other with a mixture of water and methanol in a ratio of 1:0.7 to 1:1.3 by volume. That is, filter cakes were dissolved in water and methanol, and filtered. The insoluble fraction is then recrystallized to obtain the pure crystalline cucurbituril derivative with n=7 in the formula (1). Also, the filtrate, which is the water and methanol soluble fraction, is recrystallized with an aqueous solution of acid to obtain the crystalline cucurbituril derivative with n=5 in the formula (1).

In the second synthetic method of the cucurbituril derivatives according to the present invention, a relatively small amount of acid than in the previously mentioned method is added to the reaction mixture of glycoluril and formaldehyde to result in an intermediate product in a gel state. Then, the intermediate product is treated with an acid to obtain the cucurbituril derivatives having the formula (1).

In particular, an acid is added to glycoluril in an amount of 0.1 to 1 moles with respect to 1 mole of glycoluril. The acid added may be diluted with water or an organic solvent in the same ratio as in the first method mentioned above.

Then, 2 to 20 moles, but preferably, 2 to 4 moles, of formaldehyde with respect to 1 mole of glycoluril is added to the mixture, and reacted at 70 to 85°C to obtain the intermediate product in a gel state.

After drying the intermediate product, 3 to 7 moles of an acid with respect to 1 mole of the intermediate product are added and stirred at 70 to 105°C. Here, the acid is diluted with water or an organic solvent prior to the addition.

In the above-described method, the final reaction product slightly varies depending on the reaction temperature and the amount of reactants, as in the first synthesis method. However, the usual final reaction product is a mixture of two or more cucurbituril derivatives in the formula (1), where n is a value from 5 to 20.

Typically, the final reaction product is a mixture of 5 to 30% of the cucurbituril derivative having n=5, 30 to 70% of the cucurbituril derivative having n=6, 5 to 30% of the cucurbituril derivative having n=7, 2 to 15% of the cucurbituril derivative having n=8, and 1 to 10% of the cucurbituril derivatives having n=9 to 20.

Then, the fractional separation is carried out to give the cucurbituril derivatives having the formula (2), where n is a value from 5 to 20, as in the first synthesis method.

The third synthetic method of the cucurbituril derivatives disclosed by the present invention involves a reaction under a high pressure condition, which is different from the two previously mentioned synthetic methods. In this synthetic method, the amount of acid added for the reaction with glycoluril and formaldehyde is reduced, as in the second synthesis method, to produce oligomer powder as an intermediate product. The obtained intermediate product is treated with an acid to synthesize the cucurbituril derivatives having the formula (1). In contrast to the two previously mentioned methods, the cucurbituril derivative having n=7 is produced in a relatively higher yield, whereas the cucurbituril derivative having n=6 is produced in a relatively lower yield.

The third synthetic method will now be described in particular. Glycoluril, 0.1 to 1 moles of an acid and 2 to 4 moles of formaldehyde are put into a high-pressure reactor. The acid is diluted with water or an organic solvent prior to use. The concentration of the diluted acid solution may vary depending on the reaction conditions, but preferably, 1 to 12M, more preferably 6 to 12M, of the acid solution is added. Then, the mixture is reacted at 80 to 130°C. Preferably, the reaction is carried out under a pressure of 15 to 100 psi, but preferably, 20 to 80 psi. If the reaction pressure is higher than 100 psi, it may place the synthesis process itself into a dangerous situation. Meanwhile, if the reaction pressure is lower than 15 psi, the yield may be low.

After the reaction is completed, the solid reaction product is washed with water or an organic solvent and dried to yield oligomers in powder form as an intermediate product.

Then, 3 to 7 moles of an acid for each mole of oligomers is added to the oligomers, and the mixture is stirred at 70 to 105°C. The acid added herein is diluted with water or an organic solvent, as in the first and second synthesis methods. The concentration and types of the useful acids are also identical to those in the first and second synthesis methods. As a result, a mixture of two or more cucurbituril derivatives, where n is a value from 5 to 20, is obtained as in the first and second synthesis methods.

Typically, the final reaction product is a mixture of 5 to 30% of the cucurbituril derivative having n=5, 30 to 70% of the cucurbituril derivative having n=6, 5 to 30% of the cucurbituril derivative having n=7, 2 to 15% of the cucurbituril derivative having n=8, and 1 to 10% of the cucurbituril derivatives having n=9 to 20.

After the reaction between the oligomers and the acid is completed, the separation procedure is carried out to give the cucurbituril derivatives having the formula (1), where n is a value from 5 to 20, as in the first and second synthesis methods.

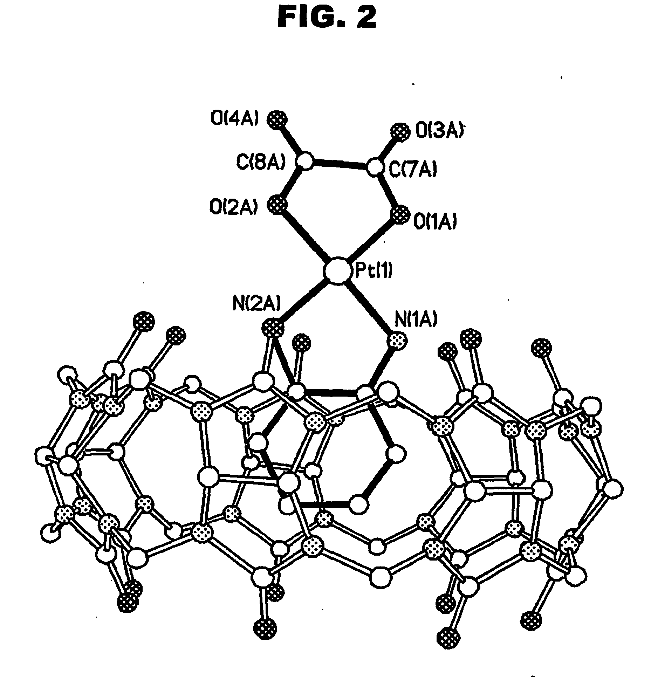

The present invention provides easy preparation methods for the cucurbituril derivatives having the formula (1), where n ranges from 5 to 11, and separation methods based on their different solubilities in a common solvent such as water, acetone and an organic solvent such as methanol. Also, according to this procedure, mixtures of at least two selected from the cucurbituril derivatives having the formula (1), where n is a value from 5 to 20. These mixtures can be separated as each cucurbituril derivative by methods shown in FIG. 2.

The pharmaceutical composition according to the present invention includes the inclusion compound of the formula 3. The inclusion compound is contained in the pharmaceutical composition in a pharmaceutically effective amount. In the case of using a platinum complex, e.g., cisplatin, as the metal complex of the formula 2, the inclusion compound can be advantageously used as an anticancer agent. The anticancer agent can prevents the platinum complex as an effective component from being biologically degraded in vivo and can exhibit continuous drug effect for a long time just by a single dosage by controlling the release time of the platinum complex once it reaches target tumor cells.

The pharmaceutical composition of this invention can be administered to humans or animals orally or non-orally. For example, the inclusion compound dissolved or suspended in an injection solvent such as injection water, a saline solution, 5% glucose aqueous solution, aqueous ethanol, aqueous glycerine or aqueous propylene glycol, in the form of, for example, intravenous injection, intramuscular injection, subcutaneous injection or instillation.

In the pharmaceutical composition of the invention, formulations include tablets, capsules, soft capsules, liquid preparations, powdery preparations and the like. The formulations in the state of a solution or a suspension can be preserved in sealed ampoules or vials, in the form of, for example, granules, powders, microgranules or freeze dried preparations for being dissolved directly before use. A stabilizer may be further added to the formulations.

When the pharmaceutical composition of the invention, including the inclusion compound and metal complexes such as the compounds represented by the formulas 4-27, is administered for treatment of cancer, its daily dose for adult person is substantially the same as that of an anticancer agent, for example, in an amount of approximately 2000 mg/dose, typically once a week or every 3 or 4 weeks, which can be administered orally or non-orally.

The pharmaceutical composition of the invention can be advantageously used for treatment of various types of cancers such as ovarian cancer, breast cancer or colonic cancer.

Various kinds of pharmaceutically acceptable additives can be further added to the pharmaceutical composition according to the invention, and the concentration thereof may vary depending on the addition purposes.

### Brief Description of the Drawings

FIG. 1 is a schematic diagram showing synthesis mechanism of cucurbituril derivatives of the formula 1;
FIG. 2 is a diagram of the X-ray crystal structure of an inclusion compound according to Synthesis Example 1 of the present invention; and
FIG. 3 is a titration graph of the inclusion compound according to Synthesis Example 1 of the present invention, measured by an isothermal microcalorimeter.

### Best mode for carrying out the Invention

The present invention is illustrated in more detail by the following examples without, however, being limited thereby.

### Examples

### Synthesis Example 1: Synthesis of inclusion compound of oxaliplatin and cucurbitu[7]ril

3 mg of oxaliplatin and 9 mg of cucurbitu[7[ril were added to 20 mL water and hydrothermally reacted at 100°C for 24 hours at a hydrothermal reactor with a reaction vessel made of Teflon. The reaction mixture was slowly cooled down to room temperature to give a colorless, plate-shaped crystalline material. The crystalline material was then filtered and dried in the presence of air, thereby obtaining an inclusion compound at a yield of 61 %.

### Synthesis Example 2: Synthesis of inclusion compound of oxaliplatin and cucurbitu[7]ril

3 mg of oxaliplatin and 9 mg of cucurbitu[7[ril were added to 20 mL water and reacted, and then ethanol was added to the mixture to form a precipitate. Thereafter, the precipitate was filtered and dried in the presence of air, thereby obtaining an inclusion compound at a yield of 72%.

The inclusion compounds synthesized in Synthesis Examples 1 and 2 were subjected to analysis by NMR (500 MHz), CHNS determination and Mass spectrometry, respectively, and the results thereof are:
¹H NMR (500MHz, D₂O) 2.26(2H, m), 1.96-1.98(2H, m), 1.60-1.62(2H, m), 1.21-1.23(2H, m), 1.07-1.09(2H, m)

| | | | |
|---|---|---|---|
| Elemental analysis {(C₈H₁₄N₂O₄Pt)(C₄₂H₄₂N₂₈O₁₄)}·8H₂O | | | |
| Cald. | C, 34.87; | H, 4.33; | N, 24.40 |
| Found | C, 34.47; | H, 4.43; | N, 24.65 |

ESI-MS m/z [M+2Li]²⁺ 786.7 (Cald: 787.3), [M+2Li+DMF]²⁺ 823.3 (Cald: 823.2), [M+Li]⁺ 1566.5 (Cald: 1566.5), [M+Li+Lil]⁺ 1700.5 (Cald: 1700.3), [M+Li+2LiI]²⁺ 1834.4 (cald: 1834.3), [M+Li+3LiI]³⁺ 1968.4 (Cald: 1968.1)

Also, the X-ray crystal structure of the inclusion compound was investigated, and the result thereof is shown in FIG. 2. FIG. 2 shows X-ray crystal structure of the inclusion compound, and data thereof are listed below:
C₅₀H_{81.33}N₃₀O_{31.67}Pt, m/w=1804.54, Orthorhombic, Space group *P*2₁2₁2₁, *a*=23.6543(5), *b*=30.23670(10), *c*=31.2827(6), Volume=22374.3(6), R₁=0.1093

Referring to FIG. 2, the inclusion compound contains cucurbitu[7]ril as a host molecule and oxaliplatin as a guest molecule, in a binding ratio of 1:1. According to observation of the host-guest molecular structure, an amine atom of the guest molecule, that is, oxaliplatin, was maintained at the same geometric level as an oxygen atom of the host molecule, that is, cucurbituril. Also, a hydrogen bond was established between a hydrogen atom connected to the ammine of oxaliplatin and an oxygen atom of cucurbituril, and the cyclohexyl of the oxaliplatin is encapsulated in a hydrophobic capvity. In such a manner, a stable inclusion compound was formed.

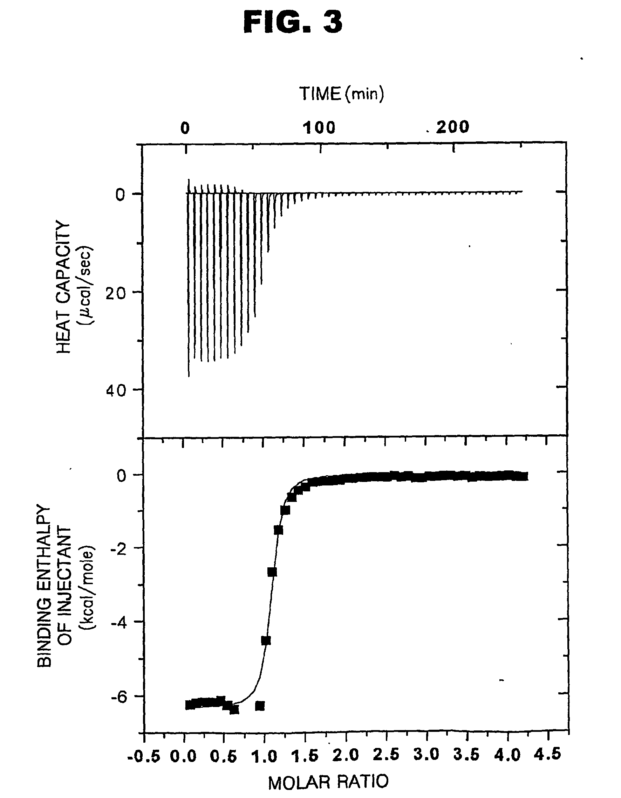

The inclusion compounds synthesized in Synthesis Examples 1 and 2 were subjected to analysis by an isothermal microcalorimeter to measure binding constants of oxaliplatin and cucurbitu[7]ril, and the results thereof are shown in FIG. 3. The experiment was carried out under a constant temperature condition of 25°C, with concentrations of 20 mM oxaliplatin and 1 mM cucurbitu[7]ril.

As a result, oxaliplatin and cucurbituril derivative had a binding constant of 2.39× 10⁵(±0.4) M⁻¹, a binding enthalpy of -6.33 kcal/mole (±0.07), and entropy of 3.31 esu. Such a relatively high binding constant of oxaliplatin and cucurbituril, compared to 60M⁻¹ of Stoddart group, suggests a very strong bond between host-guest molecules. Also, the positive entropy value confirms that the reaction is entropically advantageous as water molecule trapped by the host molecule escapes during a binding process.

### Synthesis Example 3: Synthesis of inclusion compound of oxaliplatin and cucurbitu[8]ril

13 mg of cucurbitu[8]ril and 3 mg of oxaliplatin were added to 10 mL water and hydrothermally reacted at 100°C for 24 hours, followed by slowly cooling to give a crystalline material. The crystalline material was then filtered, followed by drying in the presence of air, thereby obtaining 6 mg of an inclusion compound at a yield of 75%.

### Synthesis Example 4: Synthesis of inclusion compound of oxaliplatin and cucurbitu[8]ril

13 mg of cucurbitu[8]ril and 3 mg of oxaliplatin were added to 10 mL water and stirred at room temperature, followed by adding methanol thereto to induce precipitation. The resultant precipitate was filtered and dried in the presence of air, thereby obtaining 6 mg of an inclusion compound at a yield of 78%.

In the inclusion compounds synthesized in Synthesis Examples 3 and 4, oxaliplatin and cucurbitu[8]ril were contained in a binding ratio of 2:1.

### Synthesis Example 5: Synthesis of inclusion compound of cis-dichloroethylenediamine platinum (II) and cucurbitu[7]ril

3 mg of cis-dichloroethylenediamineplatinum (II) and 9 mg of cucurbitu[7]ril were added to 10 mL water and hydrothermally reacted at 100°C, followed by slowly cooling down, filtering and drying in the presence of air, thereby obtaining 5 mg of an inclusion compound at a yield of 65%.

### Synthesis Example 6: Synthesis of inclusion compound of cis-dichloroethylenediamine platinum (II) and cucurbitu[7]ril

3 mg of cis-dichloroethylenediamineplatinum (II) and 9 mg of cucurbitu[7]ril were added to 10 mL water and stirred at room temperature, followed by adding methanol thereto to induce precipitation. The resultant precipitate was filtered and dried in the presence of air, thereby obtaining 5 mg of a solid inclusion compound at a yield of 65%.

### Synthesis Example 7: Synthesis of inclusion compound of cis-dichloroethylenediamine platinum (II) and cucurbitu[8]ril

3 mg of cis-dichloroethylenediamineplatinum (II) and 7 mg of cucurbitu[8]ril were added to 10 mL water and hydrothermally reacted at 100°C for 24 hours. As a result, the resultant crystalline material was filtered and dried in the presence of air, thereby obtaining 5 mg of an inclusion compound at a yield of 68%.

### Synthesis Example 8: Synthesis of inclusion compound of cis-dichloroethyldiamine platinum (II) and cucurbitu[8]ril

3 mg of *cis*-dichloroethylenediamine platinum (II) and 7 mg of cucurbitu[8]ril were added to 10 mL water and stirred at room temperature. Then, methanol was added to the reaction product to form a precipitate.

Thereafter, the precipitate was filtered and dried in the presence of air, thereby obtaining 5 mg of a solid inclusion compound at a yield of 65%.

Anticancer activities of the inclusion compounds synthesized in Synthesis Examples 1 and 2 were measured as follows.

First, the oxaliplatin-cucurbitu[7]ril inclusion compounds synthesized in Synthesis Examples 1 and 2, were freeze-dried to prepare samples. Tumor cells used were A 549 (human non-small cell lung), SKOV-3 (human ovarian), SKMEL-2 (human melanoma), XF-498 (human CNS), and HCT-15 (human colon), and analyzed by SRB (sulforhodamine B) assay. The analysis results are shown in Table 1.

**Table 1: Antiproliferative activity (ED₅₀/µ M)**

| Cell line^{a} | Inclusion compounds between oxaliplatin and CB[7]^{b} |
|---|---|
| A549 | 2.43 |
| SKOV-3 | 4.88 |
| SKMEL-2 | 18.71 |
| XF-498 | 4.94 |
| HCT-15 | 13.26 |

| | |
|---|---|
| a: A549 human non-small cell lungs; SKOV-3 human ovarian; SKMEL-2 human m elanoma; XF-498 human CNS; HCT-15 human colon, b: Good water-solubility | |

As shown in Table 1, the oxaliplatin-cucurbitu[7]ril inclusion compounds synthesized in Synthesis Examples 1 and 2 exhibited good antiproliferative activities, which is presumably due to strong coordination of oxaliplatin to cucurbitu[7]ril. Also, since the inclusion compounds have capability of slowly releasing oxaliplatin, it was confirmed that continuous drug effect could be exhibited for a long time just by a single dosage of the pharmaceutical composition.

### Industrial Applicability

The inclusion compound according to the present invention include a cucurbituril derivative of the formula 1 as a host molecule and a metal complex of the formula 2 as a guest molecule. A pharmaceutical composition having an anticancer effect can be obtained by using the inclusion compound according to the present invention. The pharmaceutical composition can prevent effective components from being biologically degraded in vivo and can exhibit continuous drug effect for a long time just by a single dosage by controlling the release time of the platinum complex once it reaches target tumor cells.

## Claims

1. A pharmaceutical composition comprising an inclusion compound having a cucurbituril derivative of the formula 1 as a host molecule and a metal complex selected from the group consisting of compounds represented by formulas 4 through 27 and *cis*-dichloroethylenediamine platinum (II) as a guest molecule: 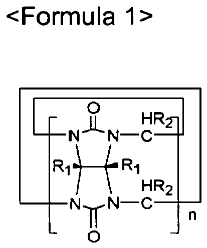 wherein R₁ and R₂ are independently selected from the group consisting of H, C₁-C₃₀ alkyl, C₁-C₃₀ alkene, C₁-C₃₀ alkyne, C₁-C₃₀ alkylthio, C₁-C₃₀ alkylcarboxy, C₁-C₃₀ alkylhydroxy, C₁-C₃₀ alkylsilyl, C₁-C₃₀ alkyloxy, C₁-C₃₀ haloalkyl, nitro, C₁-C₃₀ alkylamine, amine, C₅-C₃₀ unsubstituted cycloalkyl or C₅-C₃₀ cycloalkyl having a hetero atom, and C₆-C₁₅ unsubstituted aryl or C₆-C₁₅ aryl having a hetero atom, n is an integer from 4 to 20,

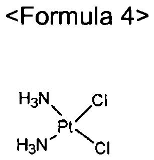

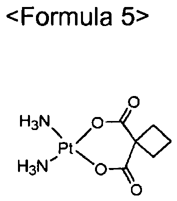

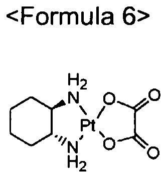

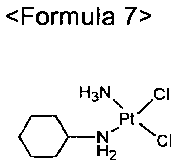

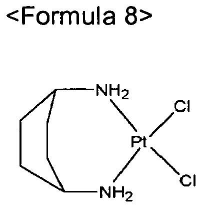

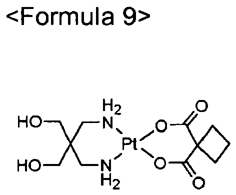

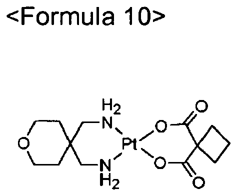

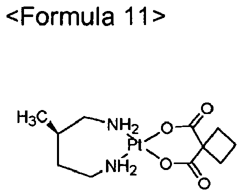

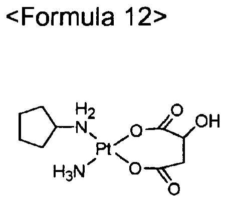

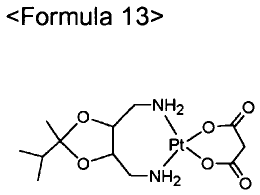

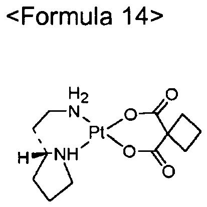

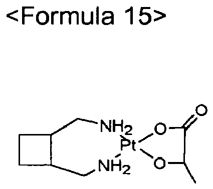

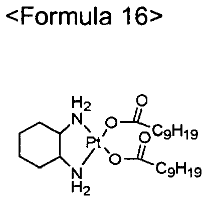

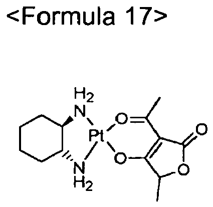

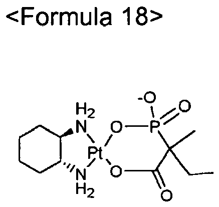

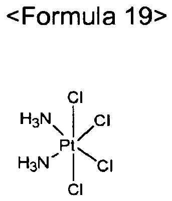

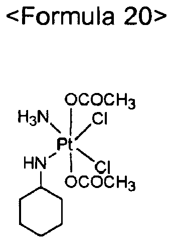

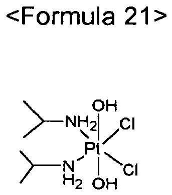

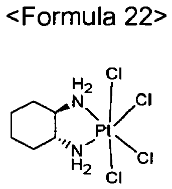

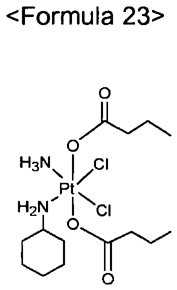

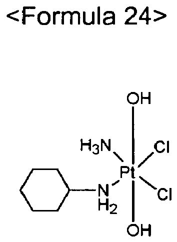

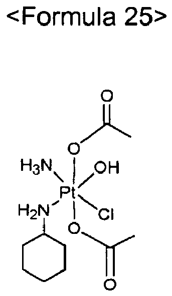

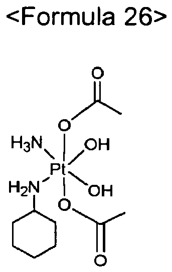

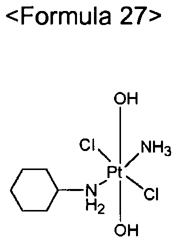

2. A pharmaceutical composition according to claim 1, wherein the binding ratio of the compound of the formula 1 to said metal complex is in the range of 1:1 to 1:8.

3. A pharmaceutical composition according to claim 1, wherein the binding ratio of the compound of the formula 1 to said metal complex is in the range of 1:1 to 1:4.

4. A pharmaceutical composition according to claim 1 wherein, in the compound of the formula 1, R₁ is hydrogen, methyl, ethyl, propyl, isopropyl, butyl, isobutyl, phenyl or pyridyl, R₂ is hydrogen, propyl, phenyl, trichloromethyl, trifluoromethyl, parafluoromethyl and α, α, α-trifluorotoluyl, and n is an integer from 5 to 8.

5. A method of preparing a pharmaceutical composition according to any of claims 1 through 4, comprising the step of reacting the cucurbituril derivative of the formula 1 with the metal complex selected from the group consisting of compounds represented by formulas 4 through 27 and *cis-*dichloroethylenediamine platinum (II).

6. A method according to claim 5, wherein the reacting step is performed at room temperature in the presence of a water solvent and includes adding an alcoholic solvent to the resultant product to separate a precipitate.

7. A method according to claim 5, wherein the reacting step is performed at a hydrothermal reactor at a temperature of 80 to 200°C, followed by cooling down to room temperature to separate a crystalline material.

8. A pharmaceutical composition according to any of claims 1 to 4 , wherein said metal complex is an anticancer active component and said inclusion compound is used for treatment of cancer.

9. A pharmaceutical composition according to any of claims 1 to 4 , wherein said inclusion compound is used for treatment of ovarian cancer, breast cancer or colonic cancer.

## Patentansprüche

1. Pharmazeutische Zusammensetzung, die eine Einschlussverbindung umfasst, welche ein Cucurbituril-Derivat der Formel 1 als ein Wirtsmolekül und einen Metallkomplex, der aus der Gruppe ausgewählt ist, die aus den Verbindungen besteht, die durch die Formeln 4 bis 27 und cis-Dichloro(ethylendiamin)-platin(II) dargestellt werden, als ein Gastmolekül aufweist. 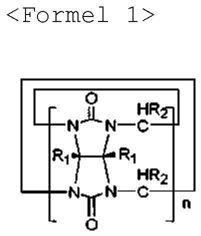 wobei R₁ und R₂ unabhängig voneinander ausgewählt sind aus der Gruppe, die aus H, C₁-C₃₀-Alkyl, C₁-C₃₀-Alken, C₁-C₃₀-Alkyn, C₁-C₃₀-Alkylthio, C₁-C₃₀-Alkylcarboxy, C₁-C₃₀-Alkylhydroxy, C₁-C₃₀-Alkylsilyl, C₁-C₃₀-Alkyloxy, C₁-C₃₀-Haloalkyl, Nitro, C₁-C₃₀-Alkylamin, Amin, C₅-C₃₀-unsubstituiertes Cycloalkyl oder C₅-C₃₀-Cycloalkyl, das ein Heteroatom aufweist, und C₆-C₁₅-unsubstituiertes Aryl oder C₆-C₁₅-Aryl, das ein Heteroatom aufweist, besteht, wobei n eine ganze Zahl von 4 bis 20 ist.

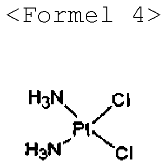

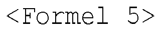

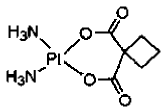

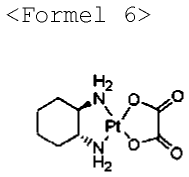

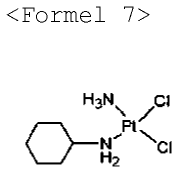

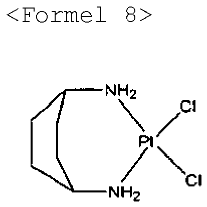

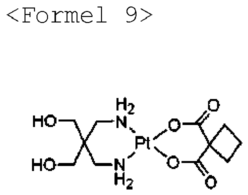

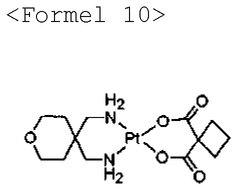

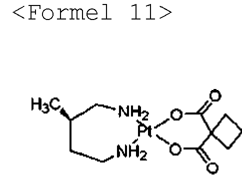

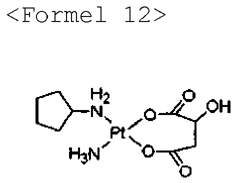

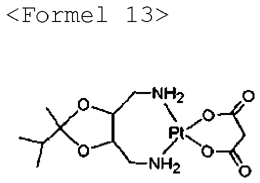

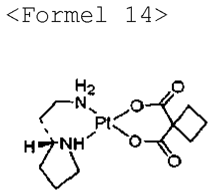

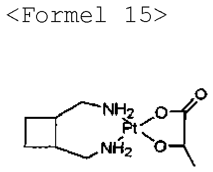

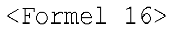

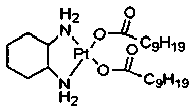

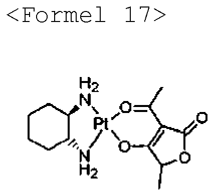

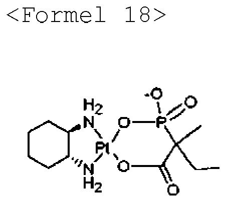

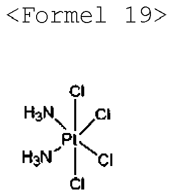

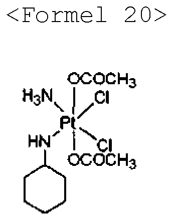

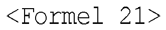

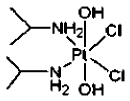

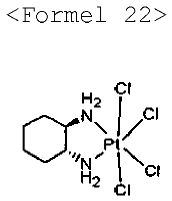

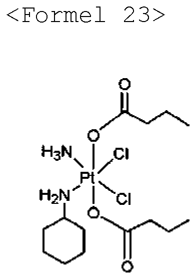

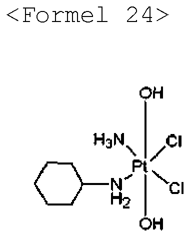

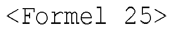

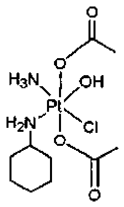

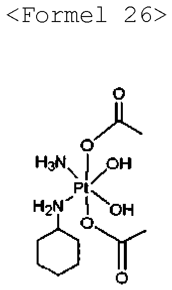

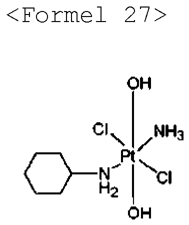

2. Pharmazeutische Zusammensetzung nach Anspruch 1, wobei das Bindungsverhältnis der Verbindung der Formel 1 mit dem Metallkomplex in einem Bereich von 1:1 bis 1:8 liegt.

3. Pharmazeutische Zusammensetzung nach Anspruch 1, wobei das Bindungsverhältnis der Verbindung der Formel 1 mit dem Metallkomplex in einem Bereich von 1:1 bis 1:4 liegt.

4. Pharmazeutische Zusammensetzung nach Anspruch 1, wobei in der Verbindung der Formel 1 R₁ für Wasserstoff,
Methyl, Ethyl, Propyl, Isopropyl, Butyl, Isobutyl, Phenyl oder Pyridyl steht, und R₂ für Wasserstoff, Propyl, Phenyl, Trichlormethyl, Trifluormethyl, para-Fluormethyl und α,α,α-Trifluortoluyl steht und n eine ganze Zahl von 5 bis 8 ist.

5. Verfahren zur Herstellung einer pharmazeutischen Zusammensetzung nach einem der Ansprüche 1 bis 4, das den Schritt der Umsetzung des Cucurbituril-Derivats der Formel 1 mit dem Metallkomplex umfasst, der ausgewählt ist aus der Gruppe, die aus den Verbindungen besteht, die durch die Formeln 4 bis 27 und *cis*-Dichloro(ethylendiamin)-platin(II) dargestellt werden.

6. Verfahren nach Anspruch 5, wobei der Umsetzungsschritt in der Anwesenheit eines wässrigen Lösemittels bei Raumtemperatur durchgeführt wird und das Hinzufügen von einem alkoholischen Lösemittel zu dem sich ergebenden Produkt beinhaltet, um dieses als einen Niederschlag abzutrennen.

7. Verfahren nach Anspruch 5, wobei der Umsetzungsschritt in einem hydrothermalen Synthese-Reaktor bei einer Temperatur von 80 bis 200 °C durchgeführt wird, gefolgt durch Abkühlen auf Raumtemperatur, um ein kristallines Material abzutrennen.

8. Pharmazeutische Zusammensetzung nach einem der Ansprüche 1 bis 4, wobei der Metallkomplex eine als Antikrebsmittel aktive Komponente ist und die Einschlussverbindung für die Behandlung von Krebs verwendet wird.

9. Pharmazeutische Zusammensetzung nach einem der Ansprüche 1 bis 4, wobei die Einschlussverbindung für die Behandlung von Eierstockkrebs, Brustkrebs oder Darmkrebs verwendet wird.

## Revendications

1. Composition pharmaceutique comprend un composé d'inclusion ayant un dérivé de cucurbiturile de formule 1 en tant que molécule hôte et un complexe métallique choisi dans le groupe constitué par les composés représentés par les formules 4 à 27 et du cis-dichloroéthylènediamine platine (II) en tant que molécule inclusse : 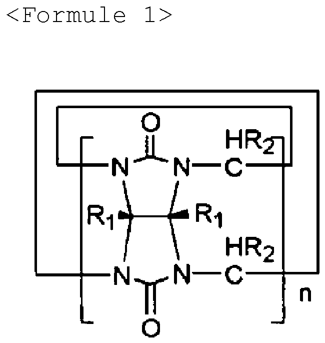 où R₁ et R₂ sont indépendamment l'un de l'autre choisi dans le groupe constitué par H, l'alkyle en C₁ à C₃₀, l'alcène en C₁ à C₃₀, l'alcyne en C₁ à C₃₀, l'alkylthio en C₁ à C₃₀, l'alkylcarboxy en C₁ à C₃₀, l'alkylhydroxy en C₁ à C₃₀, l'alkylsilyle en C₁ à C₃₀, l'alkyloxy en C₁ à C₃₀, l'haloalkyle en C₁ à C₃₀, le nitro, l'alkylamine en C₁ à C₃₀, l'amine, le cycloalkyle non substitué en C₅ à C₃₀ ou le cycloalkyle en C₅ à C₃₀ ayant un hétéroatome, et l'aryle non substitué en C₆ à C₁₅ ou l'aryle C₆ à C₁₅ ayant un hétéroatome, n est un nombre entier de 4 à 20,

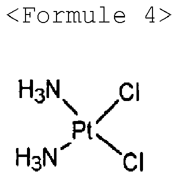

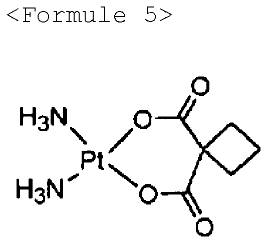

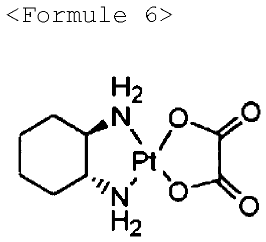

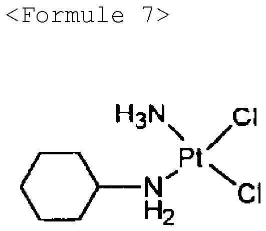

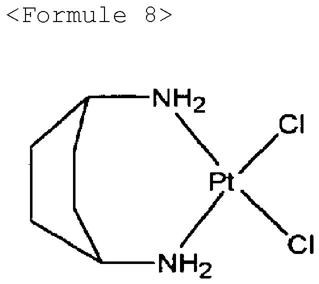

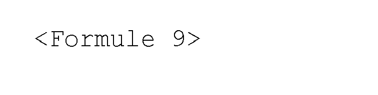

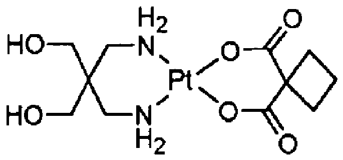

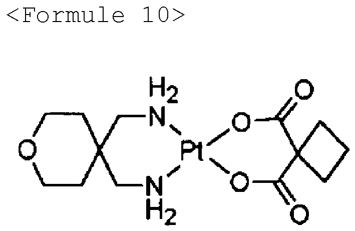

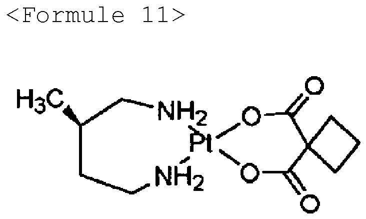

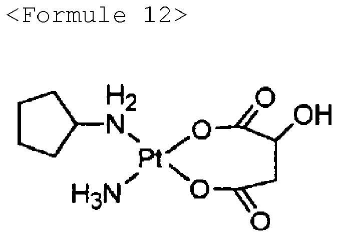

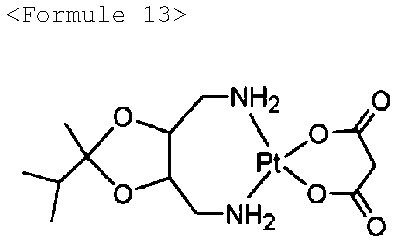

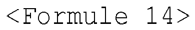

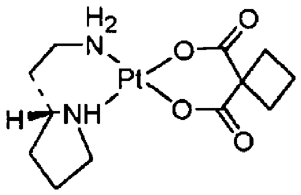

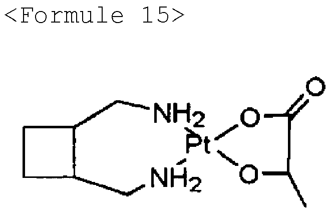

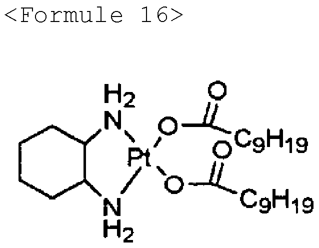

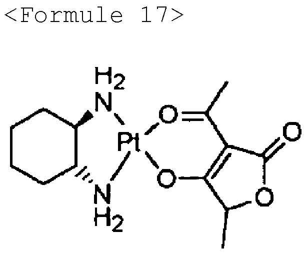

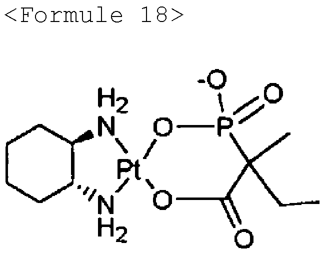

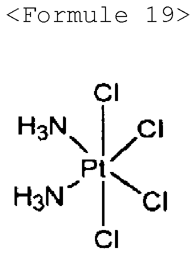

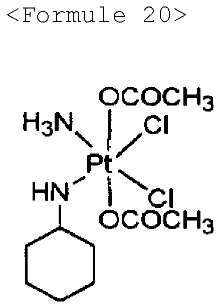

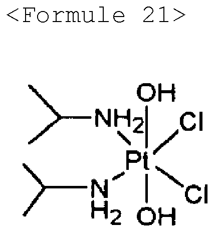

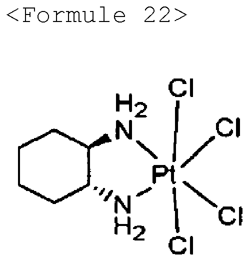

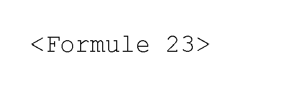

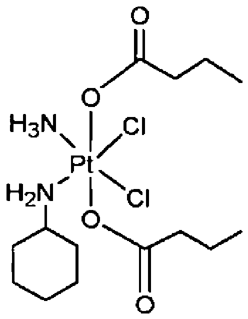

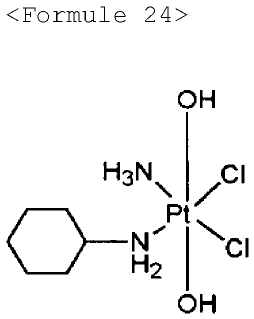

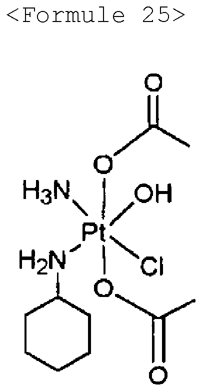

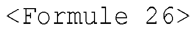

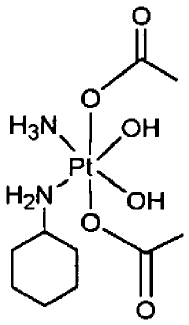

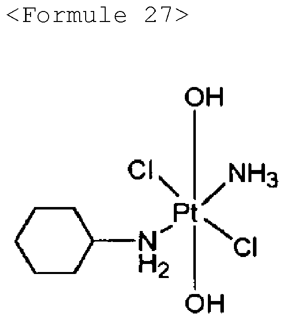

2. Composition pharmaceutique selon la revendication 1, dans laquelle le rapport de liaison du composé de formule 1 audit complexe métallique est dans la gamme de 1:1 à 1:8.

3. Composition pharmaceutique selon la revendication 1, dans laquelle le rapport de liaison du composé de formule 1 audit complexe métallique est dans la gamme de 1:1 à 1:4.

4. Composition pharmaceutique selon la revendication 1, dans laquelle, dans le composé de formule 1, R₁ est de l'hydrogène, du méthyle, de l'éthyle, du propyle, de l'isopropyle, du butyle, de l'isobutyle, du phényle ou du pyridyle, R₂ est de l'hydrogène, du propyle, du phényle, du trichlorométhyle, du trifluorométhyle, du parafluorométhyle et de l'α,α,α-trifluorotoluyle, et n est un nombre entier de 5 à 8.

5. Procédé de préparation d'une composition pharmaceutique selon l'une quelconque des revendications 1 à 4, comprenant l'étape consistant à faire réagir le dérivé de cucurbiturile de formule 1 avec le complexe métallique choisi dans le groupe constitué par les composés représentés par les formules 4 à 27 et le cis-dichloroéthylènediamine platine (II).

6. Procédé selon la revendication 5, dans lequel l'étape de réaction est effectuée à température ambiante en présence d'un solvant aqueux et comprend l'ajout d'un solvant alcoolique dans le produit obtenu pour séparer un précipité.

7. Procédé selon la revendication 5, dans lequel l'étape de réaction est effectuée dans un réacteur hydrothermique à une température de 80 à 200 °C, puis est suivie d'un refroidissement à température ambiante pour séparer un matériau cristallin.

8. Composition pharmaceutique selon l'une quelconque des revendications 1 à 4, dans laquelle ledit complexe métallique est un composant actif anticancéreux et ledit composé d'inclusion est utilisé pour le traitement du cancer.

9. Composition pharmaceutique selon l'une quelconque des revendications 1 à 4, dans laquelle ledit composé d'inclusion est utilisé pour le traitement du cancer de l'ovaire, du cancer du sein ou du cancer du côlon.
